# EUROPEAN PATENT APPLICATION

(11) **EP 4 245 377 A1**
(43) Date of publication of application: **20.09.2023**
(21) Application number: 21891825.8
(22) Date of filing: 08.11.2021
(51) Int. Cl.: A61Q 1/02, A61K 8/06, A61K 8/37, A61K 8/891

(54) **SLURRY DISPERSION, COSMETIC AND METHOD FOR PRODUCING SAME**

(30) Priority: 13.11.2020 JP 2020189448
(71) Applicant: SHIN-ETSU CHEMICAL CO., LTD., Tokyo 1000005 (JP)
(72) Inventor: KONISHI Masayuki, Tokyo 100-0005 (JP); MIYAUCHI Masaru, Tokyo 100-0005 (JP)
(74) Representative: Mewburn Ellis LLP
(86) International application number: PCT/JP2021/041010
(87) International publication number: WO 2022/102579

(57) **Abstract**

A slurry dispersion containing (A) one or more selected from trimethylsiloxysilicic acid and derivatives thereof, (B) a hydrophobized coloring pigment, and (C) an oil that is liquid at 25°C, wherein blending with a cosmetic provides a good feel on use (light feel), good applicability (spread), and an excellent feeling of adhesion and suppresses color unevenness by improving pigment dispersibility.

## Description

### TECHNICAL FIELD

The present invention relates to a slurry dispersion formulated with trimethylsiloxysilicate, a cosmetic containing the dispersion, and a method for producing the cosmetic.

### BACKGROUND ART

Trimethylsiloxysilicate, which is commonly used as a film-forming agent in cosmetics, is formulated for such purposes as to improve the staying power of the cosmetic. Patent Document 1 discloses trimethylsiloxysilicates which have modifying groups such as polyether, polyglycerol and silicone groups. Although the oil resistance and other staying power effects of trimethylsiloxysilicates are investigated herein, no research has been done on the dispersibility of coloring pigments.

Patent Document 2 describes art which, by including trimethylsiloxysilicate in makeup cosmetics, improves the finish of the makeup following application. However, the purpose of the trimethylsiloxysilicate used here is to enhance the finish of the makeup following application; no research has been done on improving the color segregation or dispersibility of coloring pigments. Also, makeup cosmetics such as foundation need to be easy to use, easy to apply and adhere well when applied to the skin; at the same time, there is a desire for such cosmetics to have a highly aesthetic quality that is free of color segregation and color irregularity.

### PRIOR ART DOCUMENTS

### PATENT DOCUMENTS

Patent Document 1: WO 2019/107497 A1
Patent Document 2: JP-A 2018-188411

### SUMMARY OF INVENTION

### TECHNICAL PROBLEM

In light of the above circumstances, the object of the present invention is to provide a slurry dispersion which, when formulated in a cosmetic, provides the cosmetic with a good feel on use (light feel), good applicability (spread) and excellent sense of adherence, and which moreover improves pigment dispersibility, thus suppressing color irregularity. Further objects of the invention are to provide a cosmetic formulated with such a slurry dispersion, and a method for producing the cosmetic.

### SOLUTION TO PROBLEM

In cosmetics colored by the formulation of pigments, color segregation arises due to agglomeration, precipitation and separation of the pigments, sometimes worsening the stability of the cosmetic over time, causing a loss in aesthetic quality and lowering the commercial value. When the amount of surfactant, etc. included is increased so as to prevent pigment agglomeration and enhance dispersibility, this sometimes leads to problems with the feel on use, such as a heavy, sticky sensation, making it difficult to satisfy both the feel on use and dispersibility at the same time. Such a phenomenon is most pronounced in emulsion-type cosmetics such as the cream foundations and liquid foundations used as base makeups. Moreover, in these base makeups, compared with point makeups, the surface area of application is large, and so importance is also placed on the ease of application (e.g., ease of spread) and adherence of the cosmetic to the skin.

The inventors have conducted intensive investigations aimed at achieving this object and have discovered as a result that the above issues can be resolved by using a slurry dispersion which includes (A) one or more compound selected from trimethylsiloxysilicate and derivatives thereof, (B) a hydrophobized coloring pigment and (C) an oil that is liquid at 25°C. This discovery ultimately led to the present invention.

Accordingly, the invention provides:
1. A slurry dispersion containing:
   (A) one or more compound selected from trimethylsiloxysilicate and derivatives thereof,
   (B) a hydrophobized coloring pigment, and
   (C) an oil that is liquid at 25°C.
2. The slurry dispersion of 1 above, wherein the component (A) content is from 0.1 to 10% by weight, the component (B) content is from 20 to 89.9% by weight and the component (C) content is from 10 to 40% by weight.
3. The slurry dispersion of 1 or 2 above, wherein component (C) is an oil selected from hydrocarbons and esters that are liquid at 25°C.
4. The slurry dispersion of any one of 1 to 3 above, wherein the weight ratio (A)/(B) of component (A) to component (B) is from 0.005 to 0.05.
5. A cosmetic which includes from 1 to 30% by weight of the slurry dispersion of any of 1 to 4 above.
6. The cosmetic of 5 above, wherein the cosmetic is a water-in-oil type emulsified cosmetic and further comprises (D) 1.5% by weight or less of an organically modified clay mineral.
7. The cosmetic of 6 above, wherein the component (D) content is from 0.1 to 0.8% by weight of the overall cosmetic.
8. A method for producing a water-in-oil type emulsified cosmetic, which method includes the steps of:
   (I) preparing a slurry dispersion by mixing together (A) one or more compound selected from trimethylsiloxysilicate and derivatives thereof, (B) a hydrophobized coloring pigment, and (C) an oil that is liquid at 25°C; and
   (II-1) mixing the slurry dispersion with an oil-phase component to form a mixture II-1, and then mixing an aqueous-phase component therein; or
   (II-2) mixing an oil-phase component with an aqueous-phase component to form a mixture II-2, and then mixing the slurry dispersion therein.

### ADVANTAGEOUS EFFECTS OF INVENTION

This invention makes it possible to provide slurry dispersions which have a good feel on use (light feel), good ease of application (spread) and excellent sense of adherence, and which moreover, by enhancing pigment dispersibility, suppress color irregularities. The invention also enables cosmetics formulated with such dispersions, and methods for producing such cosmetics, to be provided.

### DESCRIPTION OF EMBODIMENTS

The invention is described in detail below. Ingredient names in the invention are those cited in Cosmetics Labeling Nomenclature or the International Nomenclature of Cosmetic Ingredients (INCI).

### I. Slurry Dispersion

The slurry dispersion of the invention includes (A) one or more compound selected from trimethylsiloxysilicate and derivatives thereof, (B) a hydrophobized coloring pigment, and (C) an oil that is liquid at 25°C.

### [Component (A)]

Component (A) of the invention is one or more compound selected from trimethylsiloxysilicate and derivatives thereof. One such compound may be used alone or two or more may be used in combination. Component (A) enhances the dispersibility of component (B), a hydrophobized coloring pigment, in cosmetics and suppresses color segregation, thereby having the effect of not allowing the stability of the cosmetic to worsen over time so that the aesthetic quality is lost.

The trimethylsiloxysilicate (INCI name) is not particularly limited, so long as it can be used in ordinary cosmetics. This is a compound with a crosslinked structure in which a siloxane structure serves as the backbone, and is preferably one represented by the average empirical formula [(CH₃)₃SiO_{1/2}]ₐ[SiO₂]_{b} (wherein 'a' is from 1 to 3, and 'b' is from 0.5 to 8). Trimethylsiloxysilicate derivatives in which some of the (CH₃) groups in [(CH₃)₃SiO_{1/2}]ₐ are substituted with polyoxyalkyl ether, glycerol or dimethylpolysiloxane may be used. When a trimethoxysiloxysilicate derivative is used, the dispersibility sometimes improves, but the feel on use may become heavy. In cases where a good (light) feel on use is desired, trimethylsiloxysilicate is preferred.

Component (A) has a weight-average molecular weight which, for the purpose of enhancing pigment dispersibility, is preferably from 1,000 to 20,000, more preferably from 1,000 to 10,000, and even more preferably from 1,000 to 3,000. In this invention, the weight-average molecular weight is a value obtained by gel permeation chromatography (GPC) using a polystyrene standard.

### [Measurement Conditions]

| | |
|---|---|
| Developing solvent: | tetrahydrofuran (THF) |
| Flow rate: | 0.6 mL/min |
| Detector: | differential refractive index detector (RI) |
| Columns: | TSK Guardcolumn SuperH-Hone TSKgel SuperHM-N column (6.0 mm I.D. × 15 cm) one TSKgel SuperH2500 column (6.0 mm I.D. × 15 cm) (both from Tosoh Corporation) |
| Column temperature: | 40°C |
| Sample injection rate: | 50 µL (THF solution having 0.3% by weight concentration) |

Component (A) may be in the form of a liquid, gum, paste, solid or the like at 25°C. However, from the standpoint of the color irregularity-improving effects, it is preferably liquid. Also, from the standpoint of the handleability during formulation, component (A) may be diluted with a solvent and used as a solution or dispersion. The diluting solvent is preferably one or more selected from dimethylpolysiloxane, decamethylcyclopentasiloxane and isododecane. Commercial products that may be used include KF-7312J (a mixture/solution of 50% by weight decamethylcyclopentasiloxane + 50% by weight trimethylsiloxysilicate), KF-7312K (a mixture/solution of 40% by weight dimethicone (6 cs) + 60% by weight trimethylsiloxysilicate), KF-9021 (a mixture/solution of 50% by weight decamethylcyclopentasiloxane + 50% by weight trimethylsiloxysilicate), X-21-5249 (a mixture/solution of 50% decamethylcyclopentasiloxane + 50% trimethylsiloxysilicate) and X-21-5595 (a mixture of 40% by weight isododecane + 60% by weight trimethylsiloxysilicate), all of which are available from Shin-Etsu Chemical Co., Ltd. Specific examples of those which are liquid include X-21-5250 (a mixture/solution of 50% decamethylcyclopentasiloxane + 50% trimethylsiloxysilicate), X-21-5250L (a mixture/solution of 50% by weight dimethicone (6 cs) + 50% by weight trimethylsiloxysilicate) and X-21-5616 (a mixture/solution of 50% by weight isododecane + 50% by weight trimethylsiloxysilicate).

In this invention, component (A) has the advantageous effect of avoiding agglomeration of the hydrophobized coloring pigment and suppressing color segregation. In cases where, in addition to component (A), a dispersant or emulsifying agent commonly employed in cosmetics, such as a silicone-modified acrylic polymer or an alkyl polyether-modified silicone, is independently used, this effect sometimes does not appear. Because component (A) in this invention has little influence on emulsification, it may be advantageously used also in either water-in-oil type cosmetics or oil-in-water type cosmetics, although water-in-oil type cosmetics are preferred from the standpoint of the sense of adherence.

The component (A) content is preferably from 0.1 to 10% by weight, more preferably from 0.5 to 5% by weight, and even more preferably from 0.8 to 2.5% by weight, of the slurry dispersion. At less than 0.1% by weight, sufficient dispersibility may not be obtained. At more than 10% by weight, the viscosity of the slurry dispersion may increase.

### [Component (B)]

Component (B) in the invention is a hydrophobized coloring pigment. One such pigment may be used alone or two or more may be used in combination. The coloring pigment of the hydrophobized coloring pigment according to the invention is not particularly limited, provided that it is a pigment normally used for the purpose of coloring cosmetics. Examples include red iron oxide, yellow iron oxide, white titanium oxide, black iron oxide, ultramarine, Prussian blue, manganese violet, cobalt violet, chromium hydroxide, chromium oxide, cobalt oxide, cobalt titanate, iron oxide-doped titanium oxide, iron titanate, fired (titanium/titanium oxide), lithium cobalt titanate, cobalt titanate, titanium nitride, iron hydroxide, inorganic brown pigments such as gamma iron oxide, inorganic yellow pigments such as yellow ochre, and organic pigments such as lakes of tar dyes and lakes of natural dyes. Any of these may be used.

The pigment according to this invention may have any of various particle shapes, such as spherical, approximately spherical, rod-like, spindle-like, petal-like, reed-like or amorphous shapes. So long as it is able to impart color to the cosmetic, the geometric form of the pigment is not subject to any particular limitation. From the standpoint of the hiding power, a pigment having a particle size, meaning here the mean particle diameter, in the range of 150 to 600 nm is preferred. The mean particle size in this invention refers to a value that can be obtained by the particle count-based analysis of images taken with a transmission electron microscope (TEM). At less than 150 nm, the hiding power is low and so the cosmetic may have a low coloring efficiency. At a mean particle size larger than 600 nm, the feeling on use may worsen.

The pigment according to the invention may be partially or entirely surface-treated with an inorganic compound such as alumina, aluminum hydroxide, silica or hydrous silica.

"Hydrophobization" refers herein to the surface treatment of the coloring pigment with a hydrophobizing agent The surface hydrophobizing agent for the coloring pigment according to the invention is not particularly limited, so long as it can impart hydrophobicity. Exemplary treatment agents include silicone treatment agents, waxes, paraffins, organofluorine compounds such as perfluoroalkyl phosphate esters, surfactants, amino acids such as N-acylglutamic acids, and metal soaps such as aluminum stearate and magnesium myristate. Silicone treatment agents are preferred.

Exemplary silicone treatment agents include silanes or silylating agents such as caprylyl silane (AES-3083, from Shin-Etsu Chemical Co., Ltd.) and trimethoxysilyl dimethicone; silicones such as dimethyl silicones (the KF-96A Series, from Shin-Etsu Chemical Co., Ltd.), methyl hydrogen polysiloxanes (e.g., KF-99F and KF-9901 from Shin-Etsu Chemical Co., Ltd.) and silicone-branched silicone treatment agents (e.g., KF-9908 and KF-9909 from Shin-Etsu Chemical Co., Ltd.); and silicone compounds such as acrylic silicones (e.g., KP-574 and KP-541 from Shin-Etsu Chemical Co., Ltd.). Of these, use of the silicone powder treatment agents mentioned in JP No. 3912961 is preferred. For example, even when the dispersing medium that disperses the highly hydrophobized coloring pigment according to the invention is a mixture composed of a silicone and a hydrocarbon, triethoxysilylethyl polydimethylsiloxyethyl hexyl dimethicone (KF-9909, from Shin-Etsu Chemical Co., Ltd.), which is a dimethylpolysiloxane having pendent triethoxysilyl groups, polydimethylsiloxyethyl groups and hexyl groups, can be effectively used as the surface hydrophobizing agent because it exhibits a high affinity. A single surface hydrophobizing agent may be used alone or two or more may be used in combination.

In this invention, the method for surface-treating the coloring pigment using a hydrophobizing agent is not particularly limited; such surface treatment may be carried out by a known method. Surface treatment methods can be broadly divided into dry methods and wet methods. Treatment by a dry method may be carried out using any stirrer, mill, mixer or dispersing apparatus, such as a Henschel mixer, ball mill, jet mill, kneader, planetary mixer, sand mill, attritor, ribbon blender, dispersion mixer or homogenizer, to mix/contact the coloring pigment and hydrophobizing agent used in the invention. At this time, treatment may be carried out while heating or imparting the energy of, for example, a mechanochemical force or superheated steam. Alternatively, after the coloring pigment and the hydrophobizing agent have been thoroughly mixed or contacted, treatment may be carried out by, in a separate step, heating or imparting the energy of, for example, a mechanochemical force or superheated steam. Also, when mixing or contacting the hydrophobizing agent with the coloring pigment, in order to improve the efficiency of hydrophobizing agent dispersion, the hydrophobizing agent may be dissolved or dispersed beforehand in a discretionary amount of water, solvent or supercritical fluid, and use made of a means such as spraying the resulting solution or dispersion onto the coloring pigment. Treatment by a wet method may be carried out by dispersing the coloring pigment and the hydrophobizing agent in water, a solvent or a supercritical fluid so as to effect mixture or contact, subsequently evaporating off the solvent and, in a separate step, heating or imparting the energy of, for example, a mechanochemical force or superheated steam.

Specific examples of coloring pigments used to carry out hydrophobizing surface treatment include the KTP-09 Series, especially KTP-09W, 09R, 09Y and 09B (Shin-Etsu Chemical Co., Ltd.).

The component (B) content is preferably from 20 to 89.9% by weight, more preferably from 30 to 85% by weight, and even more preferably from 70 to 85% by weight, of the slurry dispersion. At less than 20% by weight, the coloring power may be inadequate; at more than 90% by weight, agglomeration among particles of the coloring pigments may increase.

The weight ratio (A)/(B) of component (A) to component (B) in the slurry dispersion is preferably from 0.005 to 0.05, and more preferably from 0.01 to 0.03. By setting this range to from 0.005 to 0.05, the dispersing effect by component (A) on component (B) can be better achieved.

### [Component (C)]

Component (C) is an oil that is liquid at 25°C. One such oil may be used alone or two or more may be used in combination. The oil that is liquid at 25°C is not particularly limited, provided that it can be used in ordinary cosmetics. Examples include vegetable oils such as jojoba oil and olive oil, animal oils such as liquid lanolin; linear or branched hydrocarbons such as liquid paraffin, light isoparaffin, liquid isoparaffin, squalane and squalene; esters such as alcohol fatty acid esters and polyhydric alcohol fatty acid esters; and silicones such as dimethylpolysiloxane, dimethylcyclopolysiloxane and methyl phenyl polysiloxane.

Of these, low-viscosity hydrocarbons, silicones and esters are preferred. More specifically, examples of preferred hydrocarbons include isododecane, dodecane, liquid paraffin, squalane and C₁₃₋₁₅ alkanes; examples of preferred esters include isotridecyl isononanoate, isononyl isononanoate, ethylhexyl palmitate, triethylhexanoin, cetyl ethyl hexanoate, hexyl laurate and glyceryl tri(caprylate/caprate); examples of preferred silicones include dimethyl polysiloxane, decamethylcyclopentasiloxane, diphenylsiloxy phenyl trimethicone and methyl trimethicone having a viscosity at 25°C of 50 mm²/s or less.

The component (C) content in the slurry dispersion is preferably from 10 to 40% by weight, more preferably from 15 to 35% by weight, and even more preferably from 15 to 25% by weight. At less than 10 %by weight, the component (B) wettability may be inadequate; at more than 40% by weight, the shear strength may be inadequate. In particular, when dispersion is carried out on a three-roll mill, the content is preferably from 15 to 30% by weight, and more preferably from 15 to 25% by weight.

As with the optional ingredients that are subsequently mentioned as liquid oil ingredients, the oil that is liquid at 25°C which is used as component (C) may be formulated in a cosmetic for such purposes as to adjust the feel on use of the cosmetic and to impart emollience, provided that, for the sake of preparing the slurry dispersion, the slurry dispersion content specified in this invention is satisfied.

Component (C) of the invention has a large influence on the stability and feel on use of cosmetics. Cases in which silicones make up a large proportion of component (C) are desirable in terms of the compatibility with component (A), the component (B) wettability, the swellability of subsequently described component (D) and the compatibility with the other ingredients, but a sense of adherence is difficult to obtain. By lowering the silicone ratio within component (C), a sense of adherence can be obtained, but the foregoing compatibility, wettability and swellability decrease and color irregularity tends to arise. With the subsequently described production method of the invention, a good dispersibility can be obtained. In cases where a sense of adherence is desired, the non-silicone proportion in component (C) is preferably at least 30% by weight, more preferably at least 50% by weight, even more preferably at least 80% by weight, and most preferably 100 %by weight. The amount of silicone included is preferably 15% by weight or less, more preferably 6% by weight or less, and even more preferably 1% by weight or less. Including no silicone is most preferred and enables a sense of adherence and the suppression of color irregularity to both be achieved.

### [Optional Ingredients]

The subsequently described film-forming agents, powders, ultraviolet scattering agents, ultraviolet absorbers, oils that are not liquid and the subsequently described component (D) may be included in the slurry dispersion of the invention. From the standpoint of pigment dispersibility and to suppress a decrease in the component (A) concentration, it is preferable for the slurry dispersion to be composed only of components (A), (B) and (C).

### [Method for Preparing Slurry Dispersion]

The method for preparing the slurry dispersion of the invention includes (I) the step of preparing a slurry dispersion by mixing together (A) one or more compound selected from trimethylsiloxysilicate and derivatives thereof, (B) a hydrophobized coloring pigment, and (C) an oil that is liquid at 25°C

No particular limitation is imposed on the method and apparatuses used when preparing the slurry dispersion of the invention. Such preparation may be carried out by a known method. For example, various stirrers, mills, mixers and dispersing apparatuses, such as Henschel mixers, ball mills, kneaders, planetary mixers, ribbon blenders, dispersion mixers, homogenizing mixers and roll mills, may be used. From the standpoint of the mixing efficiency, mixing/stirring using a three-roll mill is especially preferred. The stirring/mixing temperature, although not particularly limited, is preferably between 20°C and 105°C, and is more preferably room temperature.

### II. Cosmetic

The cosmetic of the invention includes from 1 to 30% by weight, preferably from 4 to 25% by weight, and more preferably from 10 to 25% by weight, of the above slurry dispersion. The cosmetic may be a water-in-oil type emulsified cosmetic or an oil-in-water type emulsified cosmetic, although a water-in-oil type emulsified cosmetic is preferred in terms of the feeling of adherence, and may be in the form of, for example, a milk, cream, stick or solid. Exemplary cosmetics include the following formulated with the above cosmetic ingredients: makeup cosmetics such as makeup bases, concealers, liquid foundations, cream foundations, solid foundations, rouges, eye shadows, mascaras, eye liners, eyebrows and lipsticks; and UV protection cosmetics such as sunscreen emulsions and sunscreen creams which contain hydrophobized coloring pigments. Of these, foundations, makeup bases and sunscreens which contain a plurality of coloring pigments and are intended for application to the skin are preferred.

### [Component (D)]

The cosmetic of the invention, particularly in the case of a water-in-oil type emulsified cosmetic, preferably includes (D) an organically modified clay mineral in order to enhance the pigment dispersing stability (suppress color segregation). One organically modified clay mineral may be used alone or two or more may be used in combination. The organically modified clay mineral is not particularly limited, provided that it can be used in ordinary cosmetics, and is exemplified by cation-modified clay minerals that are substituted with quaternary ammonium ions. Specific examples include layered clay minerals such as bentonite, laponite, hectorite, montmorillonite and aluminum magnesium silicate in which, by treatment with a quaternary ammonium salt-type cationic surfactant, interlayer sodium ions within the layered clay mineral have been replaced with quaternary ammonium ions.

More specifically, dimethyl distearyl ammonium hectorite, dimethyl distearyl ammonium bentonite and benzyl dimethyl stearyl ammonium hectorite are preferred; dimethyl distearyl ammonium hectorite is more preferred. Examples of commercial products include Bentone 38, Bentone 38VCG and Bentone 27 (from Elementis).

The component (D) content, expressed as the amount within the overall cosmetic, is preferably 1.5% by weight or less. That is, component (D) need not be included if the advantageous effects of the invention are achieved without component (D). When excellent adherence to the skin and the formation of a uniform thin film on the skin is desired, the content in the overall cosmetic is preferably 1.5% by weight or less (from 0 to 1.5% by weight), and more preferably from 0.1 to 0.8% by weight. To suppress the influence of component (D) upon the feel on use, a content of 0.4% by weight or less is preferred. Even when component (D) is not included, in the case of the slurry dispersion of the invention, a good dispersibility can be obtained. When more than 1.5% by weight is included, the film that forms on the skin may become nonuniform or the stability of the cosmetic over time in terms of quality may worsen.

Various optional ingredients commonly used in cosmetics may be formulated in the cosmetic of the invention.

Examples of other optional ingredients that may be formulated include (1) oils, (2) alcoholic hydroxyl group-containing compounds, (3) surfactants, (4) powders, (5) compositions made up of a crosslinked organopolysiloxane and an oil that is liquid at room temperature, (6) film-forming agents, (7) antiperspirants, (8) antibacterial agents, and (9) other additives. These may be of one type used alone or two or more may be used in suitable combination.

### (1) Oily Ingredients

The oily ingredients may be solid, semisolid or liquid. For example, natural animal and vegetable fats and oils and semi-synthetic fats and oils, hydrocarbon oils, higher fatty acids, higher alcohols, esters, silicones other than the above organosiloxanes that are essential ingredients for achieving the advantageous effects of the invention, and fluorocarbon oils may be used.

### · Solid Oily Ingredients

In this invention, when the desire is to solidify the cosmetic, it is preferable to include a wax, hydrocarbon, ester, higher alcohol or higher fatty acid that is solid at 25°C. Oily ingredients that are solid at 25°C are ingredients have a melting point of preferably at least 40°C, and more preferably between 60°C and 110°C, and are exemplified by waxes, hydrocarbons, esters, higher alcohols and higher fatty acids. Any such ingredient that normally can be formulated in cosmetics may be used without particular limitation. Specific examples include vegetable waxes such as carnauba wax, candelilla wax, rice wax and Japan wax; animal waxes such as beeswax and spermaceti; hydrocarbon waxes such as solid paraffin, polyethylene, ceresin, ozokerite and microcrystalline wax; higher alcohols such as stearyl alcohol, behenyl alcohol and cetanol; fatty acids such as stearic acid and behenic acid; silicone waxes such as acrylic/silicone resins that are acrylic/silicone graft or block copolymers (e.g., the acrylic/silicone graft copolymers KP-561P and 562P from Shin-Etsu Chemical Co., Ltd.); and derivatives of these. One of these may be used alone or two or more may be used together.

### · Natural Animal and Vegetable Oils and Fats and Semi-Synthetic Oils and Fats

Examples of natural animal and vegetable oils and fats and semi-synthetic oils and fats include natural vegetable oils and fats such as squalane, squalene, avocado oil, linseed oil, almond oil, perilla oil, olive oil, cacao oil, kapok wax, kaya nut oil, carnauba wax, candelilla wax, refined candelilla wax, apricot kernel oil, wheat germ oil, sesame oil, rice germ oil, rice bran oil, sugarcane wax, camellia kissi oil, safflower oil, shea butter, *Paulownia fortunei* oil, cinnamon oil, jojoba wax, soybean oil, *Camellia sinensis* seed oil, *Camellia japonica* seed oil, evening primrose oil, corn oil, rapeseed oil, Japanese paulownia oil, rice bran wax, germ oil, apricot (*Prunus armenica*) kernel oil, palm oil, palm kernel oil, castor oil, sunflower oil, grapeseed oil, bayberry wax, jojoba oil, macadamia nut oil, meadowfoam seed oil, cottonseed oil, cotton wax, Japan wax, Japan wax kernel oil, montan wax, palm oil and peanut oil; natural animal (including insect-derived) fats and oils such as Chinese wax, shellac wax, liver oil, tallow, neatsfoot oil, beef bone fat, spermaceti, turtle oil, lard, horse fat, beeswax, mink oil, mutton tallow, lanolin and egg oil; and semisynthetic oils and fats such as hydrogenated castor oil, castor oil fatty acid methyl esters, hydrogenated palm oil, coconut oil fatty acid triglyceride, hydrogenated tallow, hydrogenated oils, liquid lanolin, reduced lanolin, lanolin alcohol, hard lanolin, lanolin acetate, lanolin acetate alcohol, isopropyl lanolate, polyoxyethylene lanolin alcohol ether, polyoxyethylene lanolin alcohol acetate, lanolin fatty acid polyethylene glycol and polyoxyethylene hydrogenated lanolin alcohol ether.

In the subsequently described ultraviolet absorbers, when liquid at 25°C, ethylhexyl methoxycinnamate, octyl salicylate, octocrylene and the like may be formulated as non-silicones in the cosmetic.

### (2) Compounds Having an Alcoholic Hydroxyl Group

Examples of compounds having an alcoholic hydroxyl group include lower alcohols of preferably 2 to 5 carbon atoms, such as ethanol and isopropanol, and sugar alcohols such as sorbitol and maltose. Additional examples include sterols such as cholesterol, sitosterol and lanosterol.

### (3) Surfactants

The surfactants, which are exemplified by nonionic, anionic, cationic and amphoteric surfactants, are not particularly limited. Any of these may be used so long as they are employed in ordinary cosmetics. Of these surfactants, partially crosslinked polyether-modified silicones, partially crosslinked polyglycerol-modified silicones, linear or branched polyoxyethylene-modified organopolysiloxanes, linear or branched polyoxyethylene-polyoxypropylene-modified organopolysiloxanes, linear or branched polyoxyethylene/alkyl co-modified organopolysiloxanes, linear or branched polyoxyethylene-polyoxypropylene/alkyl co-modified organopolysiloxanes, linear or branched polyglycerol-modified organopolysiloxanes and linear or branched polyglycerol/alkyl co-modified organopolysiloxanes are preferred. In these surfactants, the content of hydrophilic polyoxyethylene groups, polyoxyethylene-polyoxypropylene groups or polyglycerol residues is preferably from 10 to 70% by weight of the molecule. When a partially crosslinked polyether-modified silicone or a partially crosslinked polyglycerol-modified silicone is used, in compositions made up of this crosslinked organopolysiloxane and an oil that is liquid at room temperature, it is preferable for the crosslinked organopolysiloxane to include at least its own weight of the liquid oil and to swell with respect to the liquid oil. The liquid silicones, hydrocarbon oils, ester oils, natural animal and vegetable oils, semisynthetic oils and fluorocarbon oils in the oils serving as optional ingredients may be used as this liquid oil. Examples include 0.65 to 100 mm²/s (25°C) low-viscosity silicones; hydrocarbon oils such as liquid paraffins, squalane, isododecane and isohexadecane; glyceride oils such as trioctanoin; ester oils such as isotridecyl isononanoate, N-acylglutamic acid esters and lauroyl sarcosinates; and natural animal and vegetable oils such as macadamia nut oil. Specific examples include the following from Shin-Etsu Chemical Co., Ltd.: KSG-210, 240, 310, 320, 330, 340, 320Z, 350Z, 710, 810, 820, 830, 840, 820Z and 850Z. Specific examples of surfactants that are not crosslinked organopolysiloxanes include the following from Shin-Etsu Chemical Co., Ltd.: KF-6011, 6013, 6043, 6017, 6028, 6038, 6048, 6100, 6104, 6105 and 6106. In all cases, the surfactant content is preferably from 0.1 to 20% by weight of the overall cosmetic. At 0.1% by weight or more, the dispersing and emulsifying functions are fully achievable; a content of 20% by weight or less is preferable because there is no risk of the cosmetic having a sticky feel on use. Although no limitation is imposed on the hydrophilic-lipophilic balance (HLB) of the surfactant, a value of from 2 to 14.5 is preferable for maintaining the water resistance of the cosmetic.

### (4) Powders

Aside from the above-mentioned hydrophobized coloring pigments, exemplary powders include inorganic powders, metal powders, organic powders, and inorganic/organic composite powders. Specific examples are given below. Component (B) is excluded from these powders.

### · Inorganic Powders

Exemplary inorganic powders include fine particles made of, for example, zirconium oxide, zinc oxide, cerium oxide, magnesium oxide, barium sulfate, calcium sulfate, magnesium sulfate, calcium carbonate, magnesium carbonate, talc, cleaved talc, mica, kaolin, sericite, muscovite, synthetic mica, phlogopite, red lepidolite, biotite, lepidolite, silicic acid, silicon dioxide, fumed silica, hydrous silicon dioxide, aluminum silicate, magnesium silicate, aluminum magnesium silicate, calcium silicate, barium silicate, strontium silicate, metal salts of tungstic acid, hydroxyapatite, vermiculite, hydrite, bentonite, montmorillonite, hectorite, zeolite, ceramics, dibasic calcium phosphate, alumina, aluminum hydroxide, boron nitride and glass. Examples of inorganic pearlescent coloring pigments include pearlescent pigments such as titanium oxide-coated mica, bismuth oxychloride, titanium oxide-coated bismuth oxychloride, titanium oxide-coated talc, natural pearl essence and titanium oxide-coated coloring mica.

### · Metal Powders

Examples of metal powders include fine particles of metals such as aluminum, copper, stainless steel or silver.

### · Organic Powders

Examples of organic powders include powders made of silicones, polyamides, polyacrylic acids/polyacrylic acid esters, polyesters, polyethylene, polypropylene, polystyrene, styrene/acrylic acid copolymers, divinylbenzene/styrene copolymers, polyurethanes, vinyl resins, urea resins, melamine resins, benzoguanamine, polymethyl benzoguanamine, tetrafluoroethylene, polymethyl methacrylate, cellulose, silk, nylon, phenolic resins, epoxy resins and polycarbonates. In particular, exemplary silicones include silicone resin particles (e.g., KMP-590, 591 and 592 from Shin-Etsu Chemical Co., Ltd.) and silicone resin-coated silicone rubber powders (e.g., KSP-100, 101, 102, 105, 300, 411 and 441, from Shin-Etsu Chemical Co., Ltd.). Additional examples of organic powders include metal soaps such as zinc stearate, aluminum stearate, calcium stearate, magnesium stearate, zinc myristate, magnesium myristate, zinc cetyl phosphate, calcium cetyl phosphate and zinc sodium cetyl phosphate. Further examples of organic powders include organic dyes, such as the tar dyes Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206 and Orange No. 207; and natural dyes such as carminic acid, laccaic acid, carthamin, brazilin and crocin.

### · Inorganic/Organic Composite Powders

Examples of inorganic/organic composite powders include composite powders in which the surfaces of inorganic powder particles are coated with an organic powder by a commonly known and used method.

Powders which have been surface-treated may also be used as the above powders. From the standpoint of the water resistance of the cosmetic, the surface treatment agent in such cases is preferably one which can impart hydrophobicity. Examples of treatment agents that impart such hydrophobicity include, but are not particularly limited to, silicone treatment agents, waxes, paraffins, organofluorine compounds such as perfluoroalkyl phosphate esters, surfactants, amino acids such as N-acylglutamic acids, and metal soaps such as aluminum stearate and magnesium myristate. More preferably, examples of silicone treatment agents include silanes and silylating agents such as caprylyl silane (AES-3083, from Shin-Etsu Chemical Co., Ltd.) and trimethoxysilyl dimethicone; silicones such as dimethyl silicones (the KF-96A Series from Shin-Etsu Chemical Co., Ltd.), methyl hydrogen polysiloxanes (e.g., KF-99P and KF-9901, from Shin-Etsu Chemical Co., Ltd.) and silicone-branched silicone treatment agents (e.g., KF-9908 and KF-9909, from Shin-Etsu Chemical Co., Ltd.); and acrylic silicones (KP-574 and KP-541, from Shin-Etsu Chemical Co., Ltd.). A single hydrophobizing surface treatment agent may be used alone or two or more may be used in combination.

### (5) Compositions Made of Crosslinked Organopolysiloxanes and Oils That Are Liquid at Room Temperature

In a composition made of a crosslinked organopolysiloxane and an oil that is liquid at room temperature, the crosslinked organopolysiloxane preferably includes at least its own weight of the liquid oil and swells with respect to the liquid oil. The liquid silicones, hydrocarbon oils, ester oils, natural animal and vegetable oils, semisynthetic oils and fluorocarbon oils in the oils serving as optional ingredients may be used as this liquid oil. Examples include 0.65 to 100 mm²/s (25°C) low-viscosity silicones; hydrocarbon oils such as liquid paraffin, squalane, isododecane and isohexadecane; glyceride oils such as trioctanoin; ester oils such as isotridecyl isononanoate, N-acylglutamic acid esters and lauroyl sarcosinates; and natural animal and vegetable oils such as macadamia nut oil. Component (5) differs from Component (3) according to the invention in that it is a compound which does not have a polyether or polyglycerol structure in the molecular structure. Specific examples include the KSG Series (trade name) from Shin-Etsu Chemical Co., Ltd., especially KSG-15, 16, 016F, 19, 41, 42, 43, 44, 042Z and 045Z.

### (6) Film-Forming Agents

The film-forming agent is included primarily in order to have the advantageous effects of the cosmetic last even longer. Although not subject to any particular limitation, from the standpoint of imparting water repellency, a silicone composition is preferred. Specifically, use can be made of, for example, trimethylsiloxysilicate, acrylic silicone film-forming agents, silicone-modified norbornene and silicone-modified pullulan. The film-forming agent may be first dissolved in the oil that is liquid at room temperature, and then included in the cosmetic. The liquid silicones, hydrocarbons, esters, natural animal and vegetable oils, semi-synthetic oils and fluorocarbon oils in the oils serving as optional ingredients may be used as this liquid oil. Examples include 0.65 to 100 mm²/s (25°C) low-viscosity silicones; hydrocarbon oils such as liquid paraffins, squalane, isododecane and isohexadecane; glyceride oils such as trioctanoin; ester oils such as isotridecyl isononanoate, N-acylglutamic acid esters and lauroyl sarcosinates; and natural animal and vegetable oils such as macadamia nut oil. Specific examples include the following from Shin-Etsu Silicone Co., Ltd.: KF-7321J, which is a silicone solution of trimethylsiloxysilicate, KP-545 and KP-549, which are silicone solutions of acrylic silicone film-forming agents, NBN-30-ID, which is an isododecane solution of silicone-modified norbornene, and the isododecane solution TSPL-30-ID and silicone solution TSPL-30-D5 of silicone-modified pullulan.

### (7) Antiperspirants

In cases where the cosmetic of the invention is a deodorant, an antiperspirant may be optionally included therein. So long as the antiperspirant is an ingredient that suppresses perspiration by causing the skin to tighten, general-purpose ingredients may be widely used without particular limitation. Examples include aluminum chlorohydrate, aluminum chloride, aluminum chlorohydroxy allantoinate, aluminum salts of allantoin, tannic acid, aluminum potassium sulfate, zinc oxide, zinc p-phenolsulfonate, burnt alum, aluminum zirconium tetrachlorohydrate and aluminum zirconium trichlorohydrex glycine. In particular, antiperspirant ingredients selected from the group consisting of aluminum halides, aluminum hydroxyhalides and complexes or mixtures of these with zirconium oxyhalides and zirconyl hydroxyhalides are preferred as ingredients that elicit highly advantageous effects. These antiperspirants may be dissolved in water and added, or may be used by direct addition as a powder to a preparation. Commercial products may be used as the antiperspirant. The commercial product used may be in the form of a mixture with other ingredients. The antiperspirant content is not particularly limited, and may be suitably varied according to the content of the other ingredients. To obtain deodorants having an excellent antiperspirant effect and to obtain deodorants of reduced skin irritancy, the content thereof based on the overall deodorant is preferably in the range of 0.001 to 30% by weight, and more preferably in the range of 0.01 to 20% by weight.

### (8) Antibacterial Agents

The antibacterial agents are not particularly limited, provided they are ingredients which, by suppressing the growth of resident skin bacteria that produce body odor-causing substances, enable a deodorizing effect to be achieved. For example, antibacterial drugs such as triclosan, benzalkonium chloride, benzethonium chloride, chlorhexidine hydrochloride, chlorhexidine gluconate, halocarban, and isomethylphenol are commonly used. Substances having antibacterial effects due to essential oils or extracts from crude drugs, such as dry-distilled green tea extract, may also be included. Examples of substances that may be used as antibacterial agents having deodorizing effects, such as essential oils or extracts from crude drugs, include green tea extract, lavender extract, scutellaria root extract, *Coptis japonica* extract, barley extract, *Artemisia capillaris* extract, *Aloe arborescens* extract, *Sophora angustifolia* root extract, *Sasa veitchii* leaf extract, garlic extract, witch hazel extract, black tea extract, sage leaf extract, zanthoxylum fruit extract, ginger root extract, calamus extract, *Hedera helix* extract, *Houttuynia cordata* extract, peach fruit extract, peach leaf extract, peppermint extract, *Cnidium officinale* extract, eucalyptus leaf extract, peanut skin extract, *Ganoderma lucidum* extract and *Sanguisorba officinalis* extract. One type of plant extract may be used alone or two or more may be mixed and used together.

### (9) Other Additives

Examples of other additives include oil-soluble gelling agents, ultraviolet absorbing/scattering agents, humectants, preservatives, fragrances, salts, antioxidants, pH adjustors, chelating agents, algefacients, anti-inflammatory agents, skin beautifying ingredients (whitening agents, cell activators, skin roughness improvers, circulation promoting ingredients, skin astringents, antiseborrheic agents, etc.), vitamins, amino acids, nucleic acids, hormones and inclusion compounds.

### · Oil-Soluble Gelling Agents

Examples of oil-soluble gelling agents include metal soaps, such as aluminum stearate, magnesium stearate and zinc myristate; amino acid derivatives, such as N-lauroyl-L-glutamic acid and α,γ-di-n-butylamine; dextrin fatty acid esters, such as dextrin palmitate, dextrin stearate and dextrin 2-ethylhexanoate palmitate; sucrose fatty acid esters, such as sucrose palmitate and sucrose stearate; fructooligosaccharide fatty acid esters, such as fructooligosaccharide stearate and fructooligosaccharide 2-ethylhexanoate; and benzylidene derivatives of sorbitol, such as monobenzylidene sorbitol and dibenzylidene sorbitol.

### · Ultraviolet Absorbing/Scattering Agents

In this invention, in cases where a UV blocking effect is desired in the cosmetic, it is preferable to include an ultraviolet absorbing agent.

The ultraviolet absorbing agent is not particularly limited, provided it is an ingredient that can be formulated in ordinary cosmetics. Specific examples include homomenthyl salicylate, octocrylene, t-butylmethoxydibenzoylmethane, 4-(2-β-glucopyranosiloxy)propoxy-2-hydroxybenzophenone, octyl salicylate, diethylamino hydroxybenzoyl hexyl benzoate, oxybenzone-6, oxybenzone-9, oxybenzone-1, polysilicone-15, 1-(3,4-dimethoxyphenyl)-4,4-dimethyl-1,3-pentanedione, ethylhexyl dimethoxybenzylidene dioxoimidazolidine propionate, oxybenzone-2, terephthalylidene dicamphor sulfonic acid, 2,4,6-tris[4-(2-ethylhexyloxycarbonyl)anilino]-1,3,5-triazine, methyl bis(trimethylsiloxy)silyl isopentyl trimethoxycinnamate, drometrizole trisiloxane, 2-ethylhexyl-p-dimethylaminobenzoate, isopropyl p-methoxycinnamate, ethylhexyl methoxycinnamate, bis-ethylhexyloxyphenol methoxyphenyl triazine, oxybenzone-3, oxybenzone-4, oxybenzone-5, phenylbenzimidazolesulfonic acid and methylene bis-benzotriazolyl tetramethylbutylphenol. Also, it is possible to concomitantly use a UVA absorber (e.g., diethylamino hydroxybenzoyl hexyl benzoate) and a UVB absorber (e.g., ethylhexyl methoxycinnamate); any of these respective absorbers may be combined.

Of these, one, two or more organic ultraviolet absorbers selected from ethylhexyl methoxycinnamate, diethylamino hydroxybenzoyl hexyl benzoate, octyl salicylate, polysilicone-15, t-butylmethoxydibenzoyl methane, oxybenzone, methylene bis-benzotriazolyl tetramethylbutylphenol, bis-ethylhexyloxyphenol methoxyphenyl triazine and octocrylene are especially preferred from the standpoint of the compatibility with oils.

Examples of ultraviolet scattering agents include ultraviolet light-absorbing and scattering particles such as microparticulate titanium oxide, microparticulate iron-containing titanium oxide, microparticulate zinc oxide, microparticulate cerium oxide, and composites of these. Use can also be made of a dispersion obtained by pre-dispersing these ultraviolet light-absorbing and scattering particles in an oil. The liquid silicones, hydrocarbon oils, ester oils, natural animal and vegetable oils, semisynthetic oils and fluorocarbon oils in the oils serving as optional ingredients may be used as this oil. Examples include 0.65 to 100 mm²/s (25°C) low-viscosity silicones; hydrocarbon oils, such as liquid paraffin, squalane, isododecane and isohexadecane; glyceride oils, such as trioctanoin; ester oils, such as isotridecyl isononanoate, N-acylglutamic acid esters and lauroyl sarcosinates; and natural animal and vegetable oils, such as macadamia nut oil. Specific examples of dispersions obtained by pre-dispersing ultraviolet light-absorbing and scattering particles in an oil include the SPD Series (trade name) from Shin-Etsu Chemical Co., Ltd., especially SPD-T5, Z5, T6, Z6 and T7.

### · Humectants

Examples of humectants include glycerin, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate, polyoxyethylene methyl glucoside, polyoxypropylene methyl glucoside, egg lecithin, soy lecithin, phosphatidyl choline, phosphatidyl ethanolamine, phosphatidyl serine, phosphatidyl glycerol, phosphatidyl inositol and sphingolipids.

### · Preservatives

Examples of preservatives include alkyl esters of p-oxybenzoic acid, benzoic acid, sodium benzoate, sorbic acid, potassium sorbate and phenoxyethanol. Examples of antibacterial agents include benzoic acid, salicylic acid, carbolic acid, sorbic acid, alkyl esters of p-hydroxybenzoic acid, p-chloro-m-cresol, hexachlorophene, trichlorocarbanilide, photosensitizers and phenoxyethanol.

### · Fragrances

Exemplary fragrances include natural fragrances and synthetic fragrances. Examples of natural fragrances include fragrances isolated from the flowers, leaves, wood, pericarp, etc. of plants; and fragrances from animals such as the musk ox and civet. Examples of synthetic fragrances include hydrocarbons such as monoterpenes, alcohols such as fatty alcohols and aromatic alcohols, aldehydes such as terpene aldehydes and aromatic aldehydes, ketones such as alicyclic ketones, esters such as terpene esters, lactones, phenols, oxides, nitrogen-containing compounds and acetals.

### · Salts

Exemplary salts include inorganic salts, organic acid salts, amine salts and amino acid salts. Examples of inorganic salts include the sodium, potassium, magnesium, calcium, aluminum, zirconium and zinc salts of inorganic acids such as hydrochloric acid, sulfuric acid, carbonic acid and nitric acid; examples of organic acid salts include the salts of organic acids such as acetic acid, dehydroacetic acid, citric acid, malic acid, succinic acid, ascorbic acid and stearic acid; examples of amino salts and amino acid salts include the salts of amines such as triethanolamine and the salts of amino acids such as glutamic acid. In addition, use can also be made of the salts of hyaluronic acid and chondroitin sulfuric acid, aluminum zirconium glycine complexes, etc., and also the neutralization salts of acids and alkalis used in cosmetic formulation.

### · Antioxidants

The antioxidants are not particularly limited. Examples include carotenoids, ascorbic acid and salts thereof, ascorbyl stearate, tocopheryl acetate, tocopherol, p-t-butylphenol, butylhydroxyanisole, dibutylhydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin and quercetin. One antioxidant may be used alone, or two or more may be used together.

### · pH Adjustors

Examples of pH adjustors include lactic acid, citric acid, glycolic acid, succinic acid, tartaric acid, dl-malic acid, potassium carbonate, sodium bicarbonate and ammonium bicarbonate.

### · Chelating Agents

Examples of chelating agents include alanine, sodium edetate, sodium polyphosphate, sodium metaphosphate and phosphoric acid.

### · Algefacients

Examples of algefacients include L-menthol and camphor.

### · Anti-inflammatory Agents

Examples of anti-inflammatory agents include allantoin, glycyrrhizic acid and salts thereof, glycyrrhetinic acid and salts thereof, tranexamic acid and azulene.

### · Skin-Beautifying Ingredients

Examples of skin beautifying ingredients include whitening agents such as placental extract, arbutin, glutathione and *Saxifraga sarmentosa* extract; cell activators such as royal jelly, photosensitizers, cholesterol derivatives and calf blood extract; skin roughness improvers; circulation promoting ingredients such as nonanoic acid vanillylamide, benzyl nicotinate, β-butoxyethyl nicotinate, capsaicin, zingerone, cantharides tincture, ichthammol, caffeine, tannic acid, α-borneol, tocopheryl nicotinate, inositol hexanicotinate, cyclandelate, cinnarizine, tolazoline, acetylcholine, verapamil, cepharanthine and γ-oryzanol; skin astringents such as zinc oxide and tannic acid; and antiseborrheic agents such as sulfur and thianthrol.

### · Vitamins

Examples of vitamins include vitamin A compounds such as vitamin A oil, retinol, retinyl acetate and retinyl palmitate; vitamin B compounds such as riboflavin, riboflavin butyrate, flavin adenine nucleotide and other vitamin B2 compounds, pyridoxin hydrochloride, pyridoxine dioctanoate, pyridoxine tripalmitate and other vitamin B6 compounds, vitamin B12 and derivatives thereof, and vitamin B15 and derivatives thereof; vitamin C compounds such as L-ascorbic acid, L-ascorbyl dipalmitate, sodium L-ascorbyl 2-sulfate and dipotassium L-ascorbyl diphosphate; vitamin D compounds such as ergocalciferol and cholecalciferol; vitamin E compounds such as α-tocopherol, β-tocopherol, γ-tocopherol, dl-α-tocopheryl acetate, dl-α-tocopheryl nicotinate and dl-α-tocopheryl succinate; nicotinic acids such as nicotinic acid, benzyl nicotinate and nicotinamide; vitamin H; vitamin P; pantothenic acids such as calcium pantothenate, D-pantothenyl alcohol, pantothenyl ethyl ether and acetyl pantothenyl ethyl ether; and biotin.

### · Amino Acids

Examples of amino acids include glycine, valine, leucine, isoleucine, serine, threonine, phenylalanine, arginine, lysine, aspartic acid glutamic acid, cystine, cysteine, methionine and tryptophan.

### · Nucleic Acids

An example of a nucleic acid is deoxyribonucleic acid.

### · Hormones

Examples of hormones include estradiol and ethinylestradiol.

### · Inclusion Compounds

An example of an inclusion compound is cyclodextrin.

### [Method for Producing Cosmetics]

The method for producing the inventive cosmetic is exemplified by a method for producing a water-in-oil type emulsified cosmetic which includes the steps of:
(I) preparing a slurry dispersion by mixing together (A) one or more compound selected from trimethylsiloxysilicate and derivatives thereof, (B) a hydrophobized coloring pigment, and (C) an oil that is liquid at 25°C; and
(II-1) mixing the slurry dispersion with an oil-phase component to form a mixture II-1, and mixing an aqueous-phase component therein; or
(II-2) mixing an oil-phase component with an aqueous-phase component to form a mixture II-2, and mixing the slurry dispersion therein.

By starting with, as indicated above, (I) the step of preparing a slurry dispersion, the component (B) dispersibility is enhanced, enabling color segregation following step II-1 or II-2 to be further suppressed.

In this invention, "oil-phase component" refers to, in the resulting water-in-oil emulsion cosmetic, a component that makes up the continuous phase serving as the medium (external phase) in which the emulsified particles are dispersed and that excludes the slurry dispersion. The emulsifying agent which is included for the purpose of emulsification and which dissolves or disperses the oily ingredients also is an oil-phase component. "Aqueous-phase component" refers to ingredients that make up the dispersed phase which becomes the emulsified particles (internal phase).

In steps (II-1) and (II-2), the mixing apparatus is exemplified by, in addition to (I) the step of preparing the slurry dispersion, stirrers such as paddle or propeller mixers. The mixing temperature, although not particularly limited, is preferably between 20°C and 105°C, and is more preferably room temperature. With regard to steps (II-1) and (II-2), when the oil-phase component content is from 5 to 50% by weight, (II-1) is preferable in terms of the ease of transferring the aqueous phase; when the oil-phase component content is from 10 to 80% by weight, (II-2) is preferable in terms of the viscosity at the time of emulsification. When the desire is to limit the influence of the cosmetic ingredients on the slurry dispersion, (II-2) is preferred.

### EXAMPLES

The invention is illustrated more fully below by way of Preparation Examples, Examples and Comparative Examples, although the invention is not limited by these Examples. In the following Examples, unless noted otherwise, references to "%" in the composition signify percent by weight and references to "ratio" signify a ratio by weight. The term 'content' refers to an amount included in the formulated product. The proportion of liquid non-silicones at 25°C in component (C), the contents of components (A) to (C) in the slurry dispersion, and (A)/(B) are all ratios of pure ingredient amounts.

### · Preparation of Trimethylsiloxysilicate Derivatives of Component (A)

Trimethylsiloxysilicate derivatives (Preparation Examples 1 and 2) that can be used in cosmetics of the invention were prepared as follows.

### [Preparation Example 1]

### Preparation of Isododecane Solution (60% Solids) of Glycerol-Modified Trimethylsiloxysilicate

A reactor was charged with 1,300 g of a 50% isododecane solution of a powdered hydrosilyl group-containing organosilicon resin of average empirical formula (A1) (weight-average molecular weight, 4,480; amount of hydrogen gas evolution, 8.0 mL/g), 30.7 g of the glycerol monoalkyl ether of formula (A2), 1,300 g of 2-propanol and 0.7 g of a 0.5% 2-propanol solution of chloroplatinic acid, and the reaction was effected by heating for 6 hours at 100°C. The solvent was then driven off by heating under reduced pressure. Next, 325 g of ethanol was added, following which 6.5 g of a 5% aqueous solution of sodium hydroxide was added, thereby hydrolyzing unreacted hydrosilyl groups, after which neutralization was carried out by adding 0.8 g of concentrated hydrochloric acid. Following neutralization, 195 g of 0.01N aqueous hydrochloric acid was added, thereby hydrolyzing the allyl ether groups on the unreacted polyoxyalkylene, and neutralization was carried out with 3.3 g of 5% aqueous sodium bicarbonate. The reaction product was heated under reduced pressure to drive off the solvent and filtration was carried out, giving an isododecane solution of the organic group-modified organosilicon resin of average empirical formula (A3).

| | |
|---|---|
| Average empirical formula (A1): | (Me₃SiO_{1/2})_{27.8}(HMe₂SiO_{1/2})_{1.6}(SiO₂)_{35.3} |
| Formula (A2): | CH₂=CH-CH₂-O-(CH₂CH(OH)CH₂O)-H |
| Average empirical formula (A3): | (Me₃SiO_{1/2})_{27.8}(R₂Me₂SiO_{1/2})_{1.6}(SiO_{4/2})_{35.3} R² = -CH₂-CH₂-CH₂-O-(CH₂CH(OH)CH₂O)-H |

### [Preparation Example 2]

### Preparation of Isododecane Solution (60% Solids) of Polyether-Modified Trimethylsiloxysilicate

A reactor was charged with 1,000 g of a 50% isododecane solution of a powdered hydrosilyl group-containing organosilicon resin of average empirical formula (A4) (weight-average molecular weight, 4,210; amount of hydrogen gas evolution, 6.9 mL/g), 69.1 g of the polyoxyalkylene of formula (A5), 1,000 g of 2-propanol and 0.5 g of a 0.5% 2-propanol solution of chloroplatinic acid, and the reaction was effected by heating for 6 hours at 80°C. The solvent was then driven off by heating under reduced pressure. Next, 250 g of ethanol was added, following which 5.0 g of a 5% aqueous solution of sodium hydroxide was added, thereby hydrolyzing unreacted hydrosilyl groups, after which neutralization was carried out by adding 0.6 g of concentrated hydrochloric acid. Following neutralization, 150 g of 0.01N aqueous hydrochloric acid was added, thereby hydrolyzing the allyl ether groups on the unreacted polyoxyalkylene, and neutralization was carried out with 2.6 g of 5% aqueous sodium bicarbonate. The reaction product was heated under reduced pressure to drive off the solvent and filtration was carried out, giving a 60% isododecane solution of the organic group-modified organosilicon resin of average empirical formula (A6).

The resulting isododecane solution of this organic group-modified organosilicon resin was heated between 120°C and 130°C under reduced pressure to remove the isododecane. The product thus obtained was a solid powder and had a HLB of 1.1.

| | |
|---|---|
| Average empirical formula (A4): | (Me₃SiO_{1/2})_{25.4}(HMe₂SiO_{1/2})_{1.3}(SiO₂)_{34.3} |
| Formula (A5): | CH₂=CH-CH₂-O-(C₂H₄O)₃-CH₃ |
| Average empirical formula (A6): | (Me₃SiO_{1/2})_{25.4}(R²Me₂SiO_{1/2})_{1.3}(SiO₂)_{34.3} R² = -CH₂-CH₂-CH₂-O-(C₂H₄O)₃-CH₃ |

The invention is described in detail below by way of cosmetic formulations in the following Examples and Comparative Examples, although the invention is not limited by these Examples.

### [Examples 1 to 9, Comparative Examples 1 to 10] Water-in-Oil (W/O) Foundations

In Examples 5 to 9, W/O foundations were produced by the following method using the trimethylsiloxysilicate derivatives obtained in Preparation Examples 1 and 2. Tables 1 to 4 show the compositions in Examples 1 to 9 and Comparative Examples 1 to 10.

**[Table 1]**

| Composition (%) | | Example | Comparative Example | |
|---|---|---|---|---|
| | | 1 | 1 | 2 |
| 1 | Crosslinked polyether-modified silicone mixture ¹⁾ | 3.5 | 3.5 | 3.5 |
| 2 | Crosslinked dimethylpolysiloxane mixture ²⁾ | 5 | 5 | 5 |
| 3 | Alkyl polyether-modified silicone ³⁾ | 2 | 2 | 2 |
| 4 | Disteardimonium hectorite | 1.2 | 1.2 | 1.2 |
| 5 | Decamethylcyclopentasiloxane | 20.85 | 20.85 | 20.85 |
| 6 | Neopentyl glycol diethylhexanoate | 13 | 13 | 13 |
| 7 | Spherical silicone composite powder ⁴⁾ | 2 | 2 | 2 |
| 8 | Dimethylpolysiloxane (6 cs) | 2 | 2 | 2.25 |
| 9 | Trimethylsiloxysilicate solution ⁵⁾ | 0.25 | | |
| 10 | Silicone-modified acrylic polymer ⁶⁾ | | 0.25 | |
| 11 | Silicone-treated titanium oxide ⁷⁾ | 8.5 | 8.5 | 8.5 |
| 12 | Silicone-treated red iron oxide ⁷⁾ | 0.5 | 0.5 | 0.5 |
| 13 | Silicone-treated yellow iron oxide ⁷⁾ | 0.9 | 0.9 | 0.9 |
| 14 | Silicone-treated black iron oxide ⁷⁾ | 0.1 | 0.1 | 0.1 |
| 15 | 1,3-Butylene glycol | 5 | 5 | 5 |
| 16 | Sodium citrate | 0.2 | 0.2 | 0.2 |
| 17 | Sodium chloride | 1 | 1 | 1 |
| 18 | Purified water | 34 | 34 | 34 |
| Total | | 100.0 | 100.0 | 100.0 |
| (A)/(B) | | 0.015 | - | - |
| Proportion (%) of liquid at 25°C non-silicones in Component (C) | | 36.5 | 34.6 | 35.2 |
| Content (%) of Component (A) in slurry dispersion | | 1.2 | 1.2 | 0.0 |
| Content (%) of Component (B) in slurry dispersion | | 81.6 | 81.6 | 81.6 |
| Content (%) of Component (C) in slurry dispersion | | 17.2 | 17.2 | 18.4 |
| Feel on use | | ○ | ○ | ○ |
| Ease of application | | ○ | ○ | ○ |
| Sense of adherence | | △ | × | × |
| Pigment dispersibility | | ○ | × | ×× |

| | | | | |
|---|---|---|---|---|
| 1) KSG-210 (a mixture of 70-80% dimethylpolysiloxane + 20-30% crosslinked polyether-modified silicone), from Shin-Etsu Chemical Co., Ltd. 2) KSG-15 (a mixture of 90-96% decamethylcyclopentasiloxane + 4-10% crosslinked dimethylpolysiloxane), from Shin-Etsu Chemical Co., Ltd. 3) KF-6048, from Shin-Etsu Chemical Co., Ltd. 4) KSP-101, from Shin-Etsu Chemical Co., Ltd. 5) KF-7312K (a mixture of 40% dimethylpolysiloxane (6 cs) + 60% trimethylsiloxysilicate), from Shin-Etsu Chemical Co., Ltd. 6) KP-578, from Shin-Etsu Chemical Co., Ltd. 7) KTP-09W, R, Y and B (KF-9909-treated inorganic coloring pigments; W is white, R is red, Y is yellow, and B is black), from Shin-Etsu Chemical Co., Ltd. | | | | |

### (Production Method)

Ingredients 1 to 7 were mixed to uniformity (preparation of oil-phase component). Ingredients 15 to 18 were mixed to uniformity (preparation of aqueous-phase component) and quietly added thereto, and the whole was stirred to give an emulsion. In a separate operation, Ingredients 8 to 14 were subjected to treatment on a roll mill (preparation of slurry dispersion), then added to the emulsion and mixed therewith. The resulting mixture was filled into a predetermined container, giving a foundation.

**[Table 2]**

| Composition (%) | | Example | | | | | |
|---|---|---|---|---|---|---|---|
| | | 2 | 3 | 4 | 5 | 6 | 7 |
| 1 | Crosslinked alkyl/polyether co-modified silicone mixture ¹⁾ | 3.5 | 3.5 | 3.5 | 3.5 | 3.5 | 3.5 |
| 2 | Crosslinked alkyl-modified silicone mixture ²⁾ | 2 | 2 | 2 | 2 | 2 | 2 |
| 3 | Alkyl/polyether co-modified silicone ³⁾ | 3 | 3 | 3 | 3 | 3 | 3 |
| 4 | Disteardimonium hectorite | 0.8 | 0.8 | 0.8 | 0.8 | 0.8 | 0.8 |
| 5 | Isotridecyl isononanoate | 17.4 | 23.2 | 23.2 | 23.2 | 23.2 | 23.2 |
| 6 | Dimethylpolysiloxane (2 cs) | 5.8 | | | | | |
| 7 | Spherical silicone composite powder ⁴⁾ | 2 | 2 | 2 | 2 | 2 | 2 |
| 8 | Trimethylsiloxysilicate solution ⁵⁾ | | | | | | |
| 9 | Isotridecyl isononanoate | 2.05 | 2.05 | 2.05 | 1.8 | 2.05 | 2.05 |
| 10 | Trimethylsiloxysilicate solution ⁵⁾ | 0.25 | 0.25 | | | | |
| 11 | Trimethylsiloxysilicate solution ⁶⁾ | | | 0.25 | | | |
| 12 | Trimethylsiloxysilicate from Preparation Example 1 | | | | 0.15 | 0.25 | |
| 13 | Trimethylsiloxysilicate from Preparation Example 2 | | | | | | 0.25 |
| 14 | Silicone-modified acrylic polymer ⁷⁾ | | | | | | |
| 15 | Silicone-branched alkyl/polyether co-modified silicone ⁸⁾ | | | | | | |
| 16 | Alkyl/polyether co-modified silicone ³⁾ | | | | | | |
| 17 | Silicone-branched alkyl/polyglycerol co-modified silicone ⁹⁾ | | | | | | |
| 18 | Silicone-branched polyglycerol-modified silicon ¹⁰⁾ | | | | | | |
| 19 | Silicone-treated titanium oxide ¹¹⁾ | 8.5 | 8.5 | 8.5 | 8.5 | 8.5 | 8.5 |
| 20 | Silicone-treated red iron oxide ¹¹⁾ | 0.5 | 0.5 | 0.5 | 0.5 | 0.5 | 0.5 |
| 21 | Silicone-treated yellow iron oxide ¹¹⁾ | 0.9 | 0.9 | 0.9 | 0.9 | 0.9 | 0.9 |
| 22 | Silicone-treated black iron oxide ¹¹⁾ | 0.1 | 0.1 | 0.1 | 0.1 | 0.1 | 0.1 |
| 23 | 1,3-Butylene glycol | 5 | 5 | 5 | 5 | 5 | 5 |
| 24 | Sodium citrate | 0.2 | 0.2 | 0.2 | 0.2 | 0.2 | 0.2 |
| 25 | Sodium chloride | 1 | 1 | 1 | 1 | 1 | 1 |
| 26 | Purified water | 47 | 47 | 47 | 47.35 | 47 | 47 |
| Total | | 100.0 | 100.0 | 100.0 | 100.0 | 100.0 | 100.0 |
| (A)/(B) | | 0.015 | 0.015 | 0.015 | 0.009 | 0.015 | 0.015 |
| Proportion (%) of liquid at 25°C non-silicones in Component (C) | | 80.2 | 100.0 | 100.0 | 100.0 | 100.0 | 100.0 |
| Content (%) of Component (A) in slurry dispersion | | 1.2 | 1.2 | 1.2 | 0.8 | 1.2 | 1.2 |
| Content (%) of Component (B) in slurry dispersion | | 81.3 | 81.3 | 81.3 | 83.7 | 81.3 | 81.3 |
| Content (%) of Component (C) in slurry dispersion | | 17.5 | 17.5 | 17.5 | 15.5 | 17.5 | 17.5 |
| Feel on use | | ○ | ○ | ○ | Δ | Δ | Δ |
| Ease of application | | ○ | ○ | ○ | ○ | ○ | ○ |
| Sense of adherence | | ○ | ⊚ | ⊚ | ⊚ | ⊚ | ⊚ |
| Pigment dispersibility | | ○ | △ | ○ | ○ | ⊚ | ○ |

**[Table 3]**

| Composition (%) | | Comparative Example | | | | | |
|---|---|---|---|---|---|---|---|
| | | 3 | 4 | 5 | 6 | 7 | 8 |
| 1 | Crosslinked alkyl/polyether co-modified silicone mixture ¹⁾ | 3.5 | 3.5 | 3.5 | 3.5 | 3.5 | 3.5 |
| 2 | Crosslinked alkyl-modified silicone mixture ²⁾ | 2 | 2 | 2 | 2 | 2 | 2 |
| 3 | Alkyl/polyether co-modified silicone ³⁾ | 3 | 3 | 3 | 3 | 3 | 3 |
| 4 | Disteardimonium hectorite | 0.8 | 0.8 | 0.8 | 0.8 | 0.8 | 0.8 |
| 5 | Isotridecyl isononanoate | 17.4 | 23.2 | 23.2 | 23.2 | 23.2 | 23.2 |
| 6 | Dimethylpolysiloxane (2 cs) | 5.8 | | | | | |
| 7 | Spherical silicone composite powder ⁴⁾ | 2 | 2 | 2 | 2 | 2 | 2 |
| 8 | Trimethylsiloxysilicate solution ⁵⁾ | 0.25 | | | | | |
| 9 | Isotridecyl isononanoate | 2.05 | 2.05 | 2.05 | 2.05 | 2.05 | 2.05 |
| 10 | Trimethylsiloxysilicate solution ⁵⁾ | | | | | | |
| 11 | Trimethylsiloxysilicate solution ⁶⁾ | | | | | | |
| 12 | Trimethylsiloxysilicate from Preparation Example 1 | | | | | | |
| 13 | Trimethylsiloxysilicate from Preparation Example 2 | | | | | | |
| 14 | Silicone-modified acrylic polymer ⁷⁾ | | 0.25 | | | | |
| 15 | Silicone-branched alkyl/polyether co-modified silicone ⁸⁾ | | | 0.25 | | | |
| 16 | Alkyl/polyether co-modified silicone ³⁾ | | | | 0.25 | | |
| 17 | Silicone-branched alkyl/polyglycerol co-modified silicone ⁹⁾ | | | | | 0.25 | |
| 18 | Silicone-branched polyglycerol-modified silicon ¹⁰⁾ | | | | | | 0.25 |
| 19 | Silicone-treated titanium oxide ¹¹⁾ | 8.5 | 8.5 | 8.5 | 8.5 | 8.5 | 8.5 |
| 20 | Silicone-treated red iron oxide ¹¹⁾ | 0.5 | 0.5 | 0.5 | 0.5 | 0.5 | 0.5 |
| 21 | Silicone-treated yellow iron oxide ¹¹⁾ | 0.9 | 0.9 | 0.9 | 0.9 | 0.9 | 0.9 |
| 22 | Silicone-treated black iron oxide ¹¹⁾ | 0.1 | 0.1 | 0.1 | 0.1 | 0.1 | 0.1 |
| 23 | 1,3-Butylene glycol | 5 | 5 | 5 | 5 | 5 | 5 |
| 24 | Sodium citrate | 0.2 | 0.2 | 0.2 | 0.2 | 0.2 | 0.2 |
| 25 | Sodium chloride | 1 | 1 | 1 | 1 | 1 | 1 |
| 26 | Purified water | 47 | 47 | 47 | 47 | 47 | 47 |
| Total | | 100.0 | 100.0 | 100.0 | 100.0 | 100.0 | 100.0 |
| (A)/(B) | | 0.015 | 0.015 | 0.015 | 0.015 | 0.015 | 0.015 |
| Proportion (%) of liquid at 25°C non-silicones in Component (C) | | 80.2 | 100.0 | 100.0 | 100.0 | 100.0 | 100.0 |
| Content (%) of Component (A) in slurry dispersion | | 0 | 0 | 0 | 0 | 0 | 0 |
| Content (%) of Component (B) in slurry dispersion | | 83.0 | 81.3 | 81.3 | 81.3 | 81.3 | 81.3 |
| Content (%) of Component (C) in slurry dispersion | | 17.0 | 16.7 | 16.7 | 16.7 | 16.7 | 16.7 |
| Feel on use | | ○ | ○ | ○ | ○ | △ | ○ |
| Ease of application | | ○ | ○ | △ | ○ | ○ | ○ |
| Sense of adherence | | ○ | ○ | ○ | ○ | ○ | ○ |
| Pigment dispersibility | | × | × | ×× | × | × | ×× |

The notes in Tables 2 and 3 are as follows.
1) KSG-310 (a mixture of 65-75% mineral oil + 25-35% crosslinked alkyl/polyether-modified silicone), from Shin-Etsu Chemical Co., Ltd.
2) KSG-41A (a mixture of 70-80% mineral oil + 20-30% crosslinked alkyl-modified silicone), from Shin-Etsu Chemical Co., Ltd.
3) KF-6048, from Shin-Etsu Chemical Co., Ltd.
4) KSP-101, from Shin-Etsu Chemical Co., Ltd.
5) X-21-5595 (a mixture of 40% isododecane + 60% trimethylsiloxysilicate), from Shin-Etsu Chemical Co., Ltd.
6) X-21-5616 (a mixture of 40% isododecane + 60% trimethylsiloxysilicate), from Shin-Etsu Chemical Co., Ltd.
7) KP-578, from Shin-Etsu Chemical Co., Ltd.
8) KF-6038, from Shin-Etsu Chemical Co., Ltd.
9) KF-6105, from Shin-Etsu Chemical Co., Ltd.
10) KF-6106, from Shin-Etsu Chemical Co., Ltd.
11) KTP-09W, R, Y and B (KF-9909-treated inorganic coloring pigments; W is white, R is red, Y is yellow, and B is black), from Shin-Etsu Chemical Co., Ltd.

### (Production Method)

Ingredients 1 to 8 were mixed to uniformity (preparation of oil-phase component). Ingredients 23 to 26 were mixed to uniformity (preparation of aqueous-phase component) and quietly added thereto, and the whole was stirred to give an emulsion. In a separate operation, Ingredients 9 to 22 were subjected to treatment on a roll mill (preparation of slurry dispersion), then added to the emulsion and mixed therewith. The resulting mixture was filled into a predetermined container, giving a foundation.

**[Table 4]**

| Composition (%) | | Example | | Comparative Example | |
|---|---|---|---|---|---|
| | | 8 | 9 | 9 | 10 |
| 1 | Crosslinked alkyl/polyether co-modified silicone mixture ¹⁾ | 3.5 | 3.5 | 3.5 | 3.5 |
| 2 | Crosslinked alkyl-modified silicone mixture ²⁾ | 2 | 2 | 2 | 2 |
| 3 | Alkyl/polyether co-modified silicone ³⁾ | 3 | 3 | 3 | 3 |
| 4 | Disteardimonium hectorite | 0.4 | | 0.4 | |
| 5 | Isotridecyl isononanoate | 23.6 | 19 | 23.6 | 19 |
| 6 | Spherical silicone composite powder ⁴⁾ | 2 | 2 | 2 | 2 |
| 7 | Isotridecyl isononanoate | 2.05 | 2.05 | 2.05 | 2.05 |
| 8 | Trimethylsiloxysilicate from Preparation Example 1 | 0.25 | 0.25 | | |
| 9 | Silicone-modified acrylic polymer ⁵⁾ | | | 0.25 | 0.25 |
| 10 | Silicone-treated titanium oxide ⁶⁾ | 8.5 | 8.5 | 8.5 | 8.5 |
| 11 | Silicone-treated red iron oxide ⁶⁾ | 0.5 | 0.5 | 0.5 | 0.5 |
| 12 | Silicone-treated yellow iron oxide ⁶⁾ | 0.9 | 0.9 | 0.9 | 0.9 |
| 13 | Silicone-treated black iron oxide ⁶⁾ | 0.1 | 0.1 | 0.1 | 0.1 |
| 14 | 1,3-Butylene glycol | 5 | 5 | 5 | 5 |
| 15 | Sodium citrate | 0.2 | 0.2 | 0.2 | 0.2 |
| 16 | Sodium chloride | 1 | 1 | 1 | 1 |
| 17 | Purified water | 47 | 52 | 47 | 52 |
| Total | | 100.0 | 100.0 | 100.0 | 100.0 |
| (A)/(B) | | 0.015 | 0.015 | 0 | 0 |
| Proportion (%) of liquid at 25°C non-silicones in component (C) | | 100.0 | 100.0 | 100.0 | 100.0 |
| Content (%) of component (A) in slurry dispersion | | 1.2 | 1.2 | 0 | 0 |
| Content (%) of component (B) in slurry dispersion | | 81.3 | 81.3 | 81.3 | 81.3 |
| Content (%) of component (C) in slurry dispersion | | 17.5 | 17.5 | 16.7 | 16.7 |
| Feel on use | | ○ | ⊚ | ○ | ⊚ |
| Ease of application | | ⊚ | ⊚ | ⊚ | ⊚ |
| Sense of adherence | | ⊚ | ⊚ | ○ | ○ |
| Pigment dispersibility | | ○ | △ | × | ×× |

| | | | | | |
|---|---|---|---|---|---|
| 1) KSG-310 (a mixture of 65-75% mineral oil + 25-35% crosslinked alkyl/polyether-modified silicone), from Shin-Etsu Chemical Co., Ltd. 2) KSG-41A (a mixture of 70-80% mineral oil + 20-30% crosslinked alkyl-modified silicone), from Shin-Etsu Chemical Co., Ltd. 3) KF-6048, from Shin-Etsu Chemical Co., Ltd. 4) KSP-101, from Shin-Etsu Chemical Co., Ltd. 5) KP-578, from Shin-Etsu Chemical Co., Ltd. 6) KTP-09W, R, Y and B (KF-9909-treated inorganic coloring pigments; W is white, R is red, Y is yellow, and B is black), from Shin-Etsu Chemical Co., Ltd. | | | | | |

### (Production Method)

Ingredients 1 to 6 were mixed to uniformity (preparation of oil-phase component). In a separate operation, Ingredients 7 to 13 were subjected to treatment on a roll mill (preparation of slurry dispersion). This slurry dispersion was added to the oil-phase component and uniformly mixed therewith. Next, separately prepared Components 14 to 17 (preparation of aqueous-phase component) were quietly added thereto and stirred to form an emulsion. This was added to the foregoing emulsion and mixed therewith. The resulting emulsion was filled into a predetermined container, giving a foundation.

Usage tests were conducted by ten expert female panelists on the resulting water-in-oil (W/O) type foundations, and (1) the feel on use (degree to which the formulation has a good, light feel), (2) ease of application (ease of spread), (3) sense of adherence and (4) pigment dispersibility of each foundation were evaluated according to the following criteria. The evaluation results for Examples 1 to 9 and Comparative Examples 1 to 10 are presented in the tables.

### (Evaluation Criteria)

5 points: very good - (1), (2) and (3); no color segregation - (4)
4 points: good - (1), (2), (3); substantially no color segregation - (4)
3 points: normal - (1), (2), (3); slight color segregation - (4)
2 points: somewhat poor - (1), (2), (3); substantial color segregation - (4)
1 point: poor - (1), (2), (3); very substantial color segregation - (4)

The average score for each foundation was rated according to the following criteria.

### (Rating Criteria)

⊚: Average score was 4.5 points or more
○: Average score was at least 3.5 points but less than 4.5 points
△: Average score was at least 2.5 points but less than 3.5 points
×: Average score was at least 1.5 points but less than 2.5 points
××: Average score was less than 1.5 points

Ratings of @, ○ or △ are acceptable.

The above results demonstrate that the foundations in Examples 1 to 9, compared with those in Comparative Examples 1 to 10, suppressed color separation while retaining a good feel on use and good ease of application. In cases where the desire is to obtain a sense of adherence, color segregation due to polar oils tends to arise, but it was confirmed that color segregation is suppressed by this invention. Cases in which component (B) was first dispersed by means of components (A) and (C) and used in the form of a dispersion were effective. In addition, although formulating component (D) is effective for suppressing color segregation, this invention shows that it is possible to reduce the amount of component (D) included and obtain a good feel on use.

### [Example 10] Foundation (Water-in-Oil Emulsified Cosmetic)

A foundation formulated as shown in Table 5 was prepared.

**[Table 5]**

| Composition (%) | | Example 10 |
|---|---|---|
| 1 | Crosslinked alkyl/polyether co-modified silicone mixture ¹⁾ | 5 |
| 2 | Crosslinked alkyl-modified silicone mixture ²⁾ | 1 |
| 3 | Alkyl/polyether co-modified silicone ³⁾ | 2.5 |
| 4 | Disteardimonium hectorite | 0.7 |
| 5 | Ethylhexyl methoxycinnamate | 7 |
| 6 | Diethylamino hydroxybenzoyl hexyl benzoate | 2 |
| 7 | Diphenylsiloxyphenyl trimethicone ⁴⁾ | 9.5 |
| 8 | Trimethylsiloxysilicate solution ⁵⁾ | 2 |
| 9 | Methyl trimethicone ⁶⁾ | 5 |
| 10 | Isononyl isononanoate | 2.05 |
| 11 | Trimethylsiloxysilicate solution ⁷⁾ | 0.3 |
| 12 | Silicone-treated titanium oxide ⁸⁾ | 8.5 |
| 13 | Silicone-treated red iron oxide ⁸⁾ | 0.5 |
| 14 | Silicone-treated yellow iron oxide ⁸⁾ | 0.9 |
| 15 | Silicone-treated black iron oxide ⁸⁾ | 0.1 |
| 16 | Ethanol | 5 |
| 17 | Glycerin | 3 |
| 18 | Phenoxyethanol | 0.2 |
| 19 | Sodium citrate | 0.2 |
| 20 | Sodium chloride | 1 |
| 21 | Purified water | 43.55 |
| Total | | 100.0 |
| (A)/(B) | | 0.018 |
| Proportion (%) of liquid at 25°C non-silicones in Component (C) | | 50.7 |
| Content (%) of Component (A) in slurry dispersion | | 1.5 |
| Content (%) of Component (B) in slurry dispersion | | 81.0 |
| Content (%) of Component (C) in slurry dispersion | | 17.5 |

| | | |
|---|---|---|
| 1) KSG-320 (a mixture of 70-80% isododecane + 20-30% crosslinked alkyl/polyether-modified silicone), from Shin-Etsu Chemical Co., Ltd. 2) KSG-42A (a mixture of 75-85% isododecane + 15-25% crosslinked alkyl-modified silicone), from Shin-Etsu Chemical Co., Ltd. 3) KF-6048, from Shin-Etsu Chemical Co., Ltd. 4) KF-56A, from Shin-Etsu Chemical Co., Ltd. 5) X-21-5595 (a mixture of 40% isododecane + 60% trimethylsiloxysilicate), from Shin-Etsu Chemical Co., Ltd. 6) TMF-1.5, from Shin-Etsu Chemical Co., Ltd. 7) X-21-5616 (a mixture of 40% isododecane + 60% trimethylsiloxysilicate), from Shin-Etsu Chemical Co., Ltd. 8) KTP-09W, R, Y and B (KF-9909-treated inorganic coloring pigments; W is white, R is red, Y is yellow, and B is black), from Shin-Etsu Chemical Co., Ltd. | | |

### (Production Method)

A: Ingredients 1 to 9 were uniformly mixed together (preparation of oil-phase component).
B: Ingredients 16 to 21 were uniformly mixed together (preparation of aqueous-phase component).
C: Ingredients 10 to 15 were uniformly mixed together, and then subjected to treatment on a roll mill (preparation of slurry dispersion).
D: B was gradually added to A and emulsified, following which C was added, thereby giving a foundation.

The foundation thus obtained spread easily, was dry and non-sticky, and also had excellent pigment dispersibility.

### [Example 11] Makeup Base (Water-in-Oil Emulsion Cosmetic)

A makeup base formulated as shown in Table 6 was prepared.

**[Table 6]**

| Composition (%) | | Example 11 |
|---|---|---|
| 1 | Crosslinked alkyl/polyglycerol co-modified silicone mixture ¹⁾ | 5 |
| 2 | Crosslinked alkyl-modified silicone mixture ²⁾ | 1 |
| 3 | Silicone-branched alkyl/polyglycerol co-modified silicone ³⁾ | 2.5 |
| 4 | Disteardimonium hectorite | 1 |
| 5 | Octocrylene | 5 |
| 6 | Titanium oxide dispersion ⁴⁾ | 15 |
| 7 | Zinc oxide dispersion ⁵⁾ | 15 |
| 8 | Trimethylsiloxysilicate solution ⁵⁾ | 2 |
| 9 | Ethylhexyl palmitate | 1 |
| 10 | Trimethylsiloxysilicate solution ⁶⁾ | 0.2 |
| 11 | Silicone-treated titanium oxide ⁷⁾ | 2 |
| 12 | Silicone-treated red iron oxide ⁷⁾ | 0.3 |
| 13 | Silicone-treated yellow iron oxide ⁷⁾ | 0.7 |
| 14 | Silicone-treated black iron oxide ⁷⁾ | 0.1 |
| 15 | 1,3-Butylene glycol | 5 |
| 16 | Sorbitol | 3 |
| 17 | Ethylhexylglycerin | 0.1 |
| 18 | Sodium citrate | 0.2 |
| 19 | Magnesium sulfate | 0.5 |
| 20 | Purified water | 40.4 |
| Total | | 100.0 |
| (A)/(B) | | 0.039 |
| Proportion (%) of liquid at 25°C non-silicones in Component (C) | | 48.4 |
| Content (%) of Component (A) in slurry dispersion | | 2.8 |
| Content (%) of Component (B) in slurry dispersion | | 72.1 |
| Content (%) of Component (C) in slurry dispersion | | 25.1 |

| | | |
|---|---|---|
| 1) KSG-830 (a mixture of 75-85% isododecane + 15-25% crosslinked alkyl/polyglycerol-modified silicone), from Shin-Etsu Chemical Co., Ltd. 2) KSG-42A (a mixture of 75-85% isododecane + 15-25% crosslinked alkyl-modified silicone), from Shin-Etsu Chemical Co., Ltd. 3) KF-6105, from Shin-Etsu Chemical Co., Ltd. 4) SPD-T7, from Shin-Etsu Chemical Co., Ltd. 5) SPD-Z5, from Shin-Etsu Chemical Co., Ltd. 6) X-21-5595 (a mixture of 40% isododecane + 60% trimethylsiloxysilicate), from Shin-Etsu Chemical Co., Ltd. 7) AES-3083, from Shin-Etsu Chemical Co., Ltd. | | |

### (Production Method)

A: Ingredients 1 to 8 were uniformly mixed together (preparation of oil-phase component).
B: Ingredients 15 to 20 were uniformly mixed together (preparation of aqueous-phase component).
C: Ingredients 9 to 14 were uniformly mixed together, and then subjected to treatment on a roll mill (preparation of slurry dispersion).
D: B was gradually added to A and emulsified, following which C was added, thereby giving a makeup base.

The makeup base thus obtained spread easily, was dry and non-sticky, and also had excellent pigment dispersibility.

### [Example 12] Foundation (Water-in-Oil Emulsified Cosmetic)

A foundation formulated as shown in Table 7 was prepared.

**[Table 7]**

| Composition (%) | | Example 12 |
|---|---|---|
| 1 | Crosslinked phenyl-modified silicone mixture ¹⁾ | 5 |
| 2 | Silicone-branched alkyl/polyether co-modified silicone ²⁾ | 2.5 |
| 3 | Synthetic wax | 6 |
| 4 | Paraffin wax | 2 |
| 5 | Glyceryl tri(caprylate/caprate) | 7 |
| 6 | Dicaprylyl carbonate | 7 |
| 7 | Phenyl-modified spherical silicone composite powder ³⁾ | 0.5 |
| 8 | Long-chain alkyl-containing acrylic resin ⁴⁾ | 3 |
| 9 | Cetyl ethylhexanoate | 9.5 |
| 10 | Acrylic silicone solution ⁵⁾ | 0.2 |
| 11 | Trimethylsiloxysilicate solution ⁶⁾ | 0.2 |
| 12 | Metal soap-treated microparticulate titanium oxide | 5 |
| 13 | Silicone-treated microparticulate zinc oxide | 5 |
| 14 | Caprylyl silane-treated titanium oxide ⁷⁾ | 8.5 |
| 15 | Caprylyl silane-treated red iron oxide ⁷⁾ | 0.5 |
| 16 | Caprylyl silane-treated yellow iron oxide ⁷⁾ | 0.9 |
| 17 | Caprylyl silane-treated black iron oxide ⁷⁾ | 0.1 |
| 18 | 1,3-Butylene glycol | 5 |
| 19 | Glycerin | 3 |
| 20 | Phenoxyethanol | 0.2 |
| 21 | Sodium citrate | 0.2 |
| 22 | Sodium chloride | 1 |
| 23 | Purified water | 27.7 |
| Total | | 100.0 |
| (A)/(B) | | 0.012 |
| Proportion (%) of liquid at 25°C non-silicones in Component (C) | | 84.8 |
| Content (%) of Component (A) in slurry dispersion | | 0.4 |
| Content (%) of Component (B) in slurry dispersion | | 33.4 |
| Content (%) of Component (C) in slurry dispersion | | 32.4 |

| | | |
|---|---|---|
| 1) KSG-18A (a mixture of 80-90% diphenylsiloxy phenyl trimethicone + 10-20% crosslinked phenyl-modified silicone), from Shin-Etsu Chemical Co., Ltd. 2) KF-6038, from Shin-Etsu Chemical Co., Ltd. 3) KSP-300, from Shin-Etsu Chemical Co., Ltd. 4) KP-561P, from Shin-Etsu Chemical Co., Ltd. 5) KP-550 (a mixture of 60% isododecane + 40% acrylic silicone graft copolymer), from Shin-Etsu Chemical Co., Ltd. 6) X-21-5595 (a mixture of 40% isododecane + 60% trimethylsiloxysilicate), from Shin-Etsu Chemical Co., Ltd. 7) AES-3083, from Shin-Etsu Chemical Co., Ltd. | | |

### (Production Method)

A: Ingredients 1 to 8 were uniformly mixed together (preparation of oil-phase component).
B: Ingredients 18 to 23 were uniformly mixed together (preparation of aqueous-phase component).
C: Ingredients 9 to 17 were uniformly mixed together, and then subjected to treatment with a homogenizing mixer (preparation of slurry dispersion).
D: B was gradually added to A at 85°C and emulsified, following which C was added, thereby giving a solid foundation.

The solid foundation thus obtained spread easily, was dry and non-sticky, and also had excellent pigment dispersibility.

### [Example 13] Water-in-Oil Mascara

A water-in-oil mascara formulated as shown in Table 8 was prepared.

**[Table 8]**

| Composition (%) | | Example 13 |
|---|---|---|
| 1 | Silicone-modified pullulan solution ¹⁾ | 5 |
| 2 | Isododecane | 45 |
| 3 | Disteardimonium hectorite | 1.5 |
| 4 | Dextrin palmitate | 3 |
| 5 | Ceresin | 8 |
| 6 | Microcrystalline wax | 8 |
| 7 | Polymethylsilsesquioxane ²⁾ | 5 |
| 8 | Silicone-branched polyether co-modified silicone ³⁾ | 0.4 |
| 9 | Propylene carbonate | 0.5 |
| 10 | Isododecane | 3 |
| 11 | Trimethylsiloxysilicate solution ⁴⁾ | 0.4 |
| 12 | Silicone-treated black iron oxide ⁵⁾ | 5 |
| 13 | Silicone-treated talc ⁶⁾ | 5 |
| 14 | 1,3-Butylene glycol | 3 |
| 15 | Phenoxyethanol | 0.2 |
| 16 | Sodium citrate | 0.2 |
| 17 | Sodium chloride | 0.2 |
| 18 | Purified water | 6.6 |
| Total | | 100.0 |
| (A)/(B) | | 0.048 |
| Proportion (%) of liquid at 25°C non-silicones in Component (C) | | 100.0 |
| Content (%) of Component (A) in slurry dispersion | | 1.8 |
| Content (%) of Component (B) in slurry dispersion | | 74.6 |
| Content (%) of Component (C) in slurry dispersion | | 23.6 |

| | | |
|---|---|---|
| 1) TSPL-30-ID (a mixture of 70% isododecane + 30% silicone-modified pullulan), from Shin-Etsu Chemical Co., Ltd. 2) KMP-590, from Shin-Etsu Chemical Co., Ltd. 3) KF-6028, from Shin-Etsu Chemical Co., Ltd. 4) X-21-5595 (a mixture of 40% isododecane + 60% trimethylsiloxysilicate), from Shin-Etsu Chemical Co., Ltd. 5) KTP-09B (KF-9909-treated inorganic coloring pigment), from Shin-Etsu Chemical Co., Ltd. 6) Treated with KF-9909, from Shin-Etsu Chemical CO., Ltd. | | |

### (Production Method)

A: Ingredients 1 to 9 were uniformly mixed together at 90°C (preparation of oil-phase component).
B: Ingredients 14 to 18 were uniformly mixed together at 85°C (preparation of aqueous-phase component).
C: Ingredients 10 to 13 were uniformly mixed together, and then subjected to treatment with a dispersion mixer (preparation of slurry dispersion).
D: B was gradually added to A at 85°C and emulsified, following which C was added, thereby giving a water-in-oil mascara.

The water-in-oil mascara thus obtained spread easily, was dry and non-sticky, and also had excellent pigment dispersibility.

### [Example 14] Water-in-Oil Foundation

A water-in-oil foundation formulated as shown in Table 9 was prepared.

**[Table 9]**

| Composition (%) | | Example 14 |
|---|---|---|
| 1 | Crosslinked alkyl/polyether co-modified silicone mixture ¹⁾ | 2 |
| 2 | Crosslink alkyl-modified silicone mixture ²⁾ | 2 |
| 3 | Alkyl/polyether co-modified silicone ³⁾ | 2 |
| 4 | Silicone-branched alkyl/polyether co-modified silicone ⁴⁾ | 1 |
| 5 | Disteardimonium hectorite | 0.8 |
| 6 | Homosalate | 5 |
| 7 | Diethylamino hydroxybenzoyl hexyl benzoate | 2 |
| 8 | Ethylhexyl salicylate | 5 |
| 9 | Dodecane | 5 |
| 10 | Alkyl (C₁₂₋₁₅) benzoate | 3 |
| 11 | Isopropyl palmitate | 4 |
| 12 | Trimethylsiloxysilicate solution ⁵⁾ | 0.8 |
| 13 | Silicone-treated titanium oxide ⁶⁾ | 17 |
| 14 | Silicone-treated red iron oxide ⁶⁾ | 1 |
| 15 | Silicone-treated yellow iron oxide ⁶⁾ | 1.8 |
| 16 | Silicone-treated black iron oxide ⁶⁾ | 0.2 |
| 17 | BG | 5 |
| 18 | Allantoin | 0.2 |
| 19 | Phenoxyethanol | 0.2 |
| 20 | Sodium citrate | 0.2 |
| 21 | Sodium chloride | 0.5 |
| 22 | Purified water | 41.3 |
| Total | | 100.0 |
| (A)/(B) | | 0.024 |
| Proportion (%) of liquid at 25°C non-silicones in Component (C) | | 98.7 |
| Content (%) of Component (A) in slurry dispersion | | 1.9 |
| Content (%) of Component (B) in slurry dispersion | | 80.6 |
| Content (%) of Component (C) in slurry dispersion | | 17.4 |

| | | |
|---|---|---|
| 1) KSG-320Z (a mixture of 70-80% isododecane + 20-30% crosslinked alkyl polyether-modified silicone), from Shin-Etsu Chemical Co., Ltd. 2) KSG-042Z (a mixture of 75-85% isododecane + 15-25% crosslinked alkyl-modified silicone), from Shin-Etsu Chemical Co., Ltd. 3) KF-6048, from Shin-Etsu Chemical Co., Ltd. 4) KF-6038, from Shin-Etsu Chemical Co., Ltd. 5) KF-7312K (a mixture of 40% dimethylpolysiloxane (6 cs) + 60% trimethylsiloxysilicate), from Shin-Etsu Chemical Co., Ltd. 6) KTP-09W, R, Y and B (KF-9909-treated inorganic coloring pigments; W is white, R is red, Y is yellow, and B is black), from Shin-Etsu Chemical Co., Ltd. | | |

### (Production Method)

A: Ingredients 1 to 10 were uniformly mixed together (preparation of oil-phase component).
B: Ingredients 17 to 22 were uniformly mixed together (preparation of aqueous-phase component).
C: Ingredients 11 to 16 were uniformly mixed together, and then subjected to treatment on a roll mill (preparation of slurry dispersion).
D: C was added to A and uniformly mixed therewith, following which B was gradually added, thereby giving a foundation.

The foundation thus obtained spread easily, was dry and non-sticky, and also had excellent pigment dispersibility.

The invention is not limited to the embodiments described above, which are presented here for the purpose of illustration. Any embodiments having substantially the same constitution as the technical ideas set forth in the claims and exhibiting similar working effects are encompassed within the technical scope of the invention.

## Claims

1. A slurry dispersion comprising:
(A) one or more compound selected from trimethylsiloxysilicate and derivatives thereof,
(B) a hydrophobized coloring pigment, and
(C) an oil that is liquid at 25°C.

2. The slurry dispersion of claim 1, wherein the component (A) content is from 0.1 to 10% by weight, the component (B) content is from 20 to 89.9% by weight and the component (C) content is from 10 to 40% by weight.

3. The slurry dispersion of claim 1 or 2, wherein component (C) is an oil selected from hydrocarbons and esters that are liquid at 25°C.

4. The slurry dispersion of any one of claims 1 to 3, wherein the weight ratio (A)/(B) of component (A) to component (B) is from 0.005 to 0.05.

5. A cosmetic comprising from 1 to 30% by weight of the slurry dispersion of any one of claims 1 to 4.

6. The cosmetic of claim 5, wherein the cosmetic is a water-in-oil type emulsified cosmetic and further comprises (D) 1.5% by weight or less of an organically modified clay mineral.

7. The cosmetic of claim 6, wherein the component (D) content is from 0.1 to 0.8% by weight of the overall cosmetic.

8. A method for producing a water-in-oil type emulsified cosmetic, comprising the steps of:
(I) preparing a slurry dispersion by mixing together (A) one or more compound selected from trimethylsiloxysilicate and derivatives thereof, (B) a hydrophobized coloring pigment, and (C) an oil that is liquid at 25°C; and
(II-1) mixing the slurry dispersion with an oil-phase component to form a mixture II-1, and then mixing an aqueous-phase component therein; or
(II-2) mixing an oil-phase component with an aqueous-phase component to form a mixture II-2, and then mixing the slurry dispersion therein.
